# EUROPEAN PATENT APPLICATION

(11) **EP 1 825 809 A2**
(43) Date of publication of application: **29.08.2007**
(21) Application number: 07425091.1
(22) Date of filing: 20.02.2007
(51) Int. Cl.: A61B 5/103, A61M 35/00, A61N 1/32, B05B 11/00

(54) **System and method for administering a cosmetic composition to a subject**

(30) Priority: 20.02.2006 IT MI20060306
(71) Applicant: BC System s.r.l., 20146 Milano MI (IT)
(72) Inventor: Nizardo Chailly, Alessandro, 20037 Pademo Dugnano MI (IT)
(74) Representative: Montelatici, Linda Anna

(57) **Abstract**

The present invention relates to a system for administering a cosmetic composition to a subject (3), comprising first means (4, 4A, 4B) suitable to deduce characteristic data (A, A', A", A"') about the skin status of the subject (3), an apparatus (6) suitable to generate at least one micro-channel in an epidermis region of the subject (3) in order to facilitate the administration of a dermatologically compatible cosmetic composition (2) through said epidermis region. A characteristic is the fact of comprising second means (5, 5A, 5B, 5C) suitable to select at least one protocol (PRGᵢ, PRGⱼ) among a plurality of predefined protocols (PRG_{N}, PRG_{I}) in function of said characteristic data (A, A', A", A''') which are deduced by said first means (4, 4A, 4B) wherein said dermatologically compatible cosmetic composition (2) is selected in function of said protocol (PRGᵢ, PRGⱼ) and comprises a dermatologically compatible natural product and at least one excipient.

## Description

The present invention relates to a system and a method for administering a cosmetic composition to a subject, according to the preamble of claims 1 and 10.

In the context of the present disclosure, the term electroporation device is used to indicate a device which is suitable to generate a series of electrical pulses able to open between dermis cells one or several micro-channels - the so-called electropores - through which said electroporation device is also able to carry specific substances by using the iontophoresis principle.

For simplicity of presentation, the present disclosure is made in a non-limiting way with particular reference to treatments for the visage and for EFP (edematous fibrosclerotic panniculopathy) or cellulite, but other body zones, such as neck, décolletage, breast etc. can undergo a treatment by means of the system and the method according to the present invention.

Systems which are able to provide programs and treatments for caring for visage and/or body of a subject are known.

Such programs and treatments are usually performed by operators (aestheticians) at beauty shops or beauty parlors.

In today's beauty shops hi-tech apparatuses which are able to administer the above mentioned treatments are now widely used.

However, such apparatuses require extremely high operator skills and professionalism to be used in their proper way.

Further, the role of the aesthetician is fundamental for achieving good results in visage and/or body programs and treatments.

In fact the choice of the treatment or program to be performed, as well as the evaluation of the skin type and the individual needs of the subject is a burden which falls entirely on the aesthetician skill and professionalism.

Although the professional level of the operators has grown in recent years, it is to this day highly felt the demand for providing tools and methods which are able to guide the aesthetician in its job.

A further demand which is recently increasingly spread is about providing beauty treatments using cosmetic compositions which do not include synthetic substances.

In view of the described prior art, the object of the present invention is to solve the problems and the above mentioned demands with reference to the prior art.

According to the present invention, said object is achieved by means of a system for administering a dermatologically compatible, natural and cosmetic composition to a subject according to claim 1.

Said object is also achieved by means of a method for administering a dermatologically compatible, natural and cosmetic composition to a subject according to claim 10.

Thanks to the present invention it is possible to provide a system and a method which are able to increase the quality standard of beauty shops, so as to achieve notably better cosmetic results, client management and job quality with respect to known systems.

Further, thanks to the present invention, cosmetic products having a natural origin and a high quality and effectiveness are used.

The characteristics and the advantages of the present invention will be evident from the following detailed description of a practical embodiment, which is illustrated by way of non limiting example in the appended drawings, wherein:
- Figures 1, 2A and 2B show possible uses of the system according to the present invention;
- Figures 3A and 3B show a first and a second synoptic table according to the present invention.

With reference to the annexed figures, 1 indicates the system for administering a cosmetic composition 2 to a subject 3.

In the preferred embodiment of the present invention the system 1 comprises:
- first means 4 suitable to deduce characteristic data A about the skin status of the subject 3;
- second means 5 suitable to select at least one protocol PRGᵢ, PRGⱼ among a plurality of predefined protocols PRG_{N}, PRG_{I} in function of the characteristic data A which are deduced by the first means 4; and
- an apparatus 6 suitable to generate at least one micro-channel in a region of the epidermis 3B of the subject 3 in order to facilitate administering the cosmetic composition 2 through said region of the epidermis 3B.

Advantageously, the cosmetic composition 2 is a dermatologically compatible cosmetic composition which comprises a natural product and one or several excipients.

Said cosmetic composition can more specifically be a cosmeceutic composition.

For example the natural product is selected from one or more phytocomplexes or derivates thereof, such as for example: extensin; hop; ginseng; escin; horsetail; echinacea, etc.

The cosmetic composition 2 is thus selected in function of the selected protocol PRGᵢ, PRGⱼ, as subsequently described in more detail.

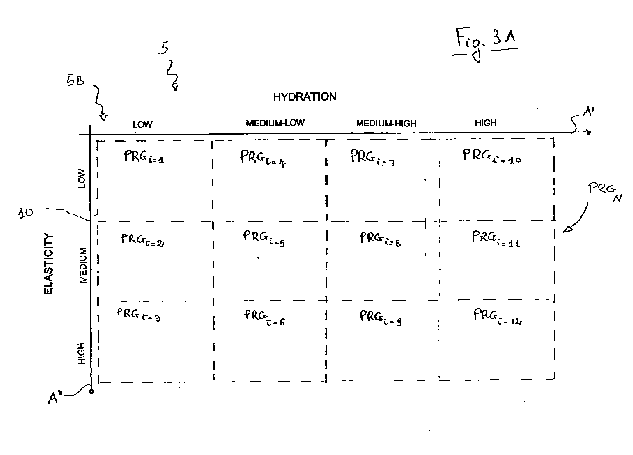

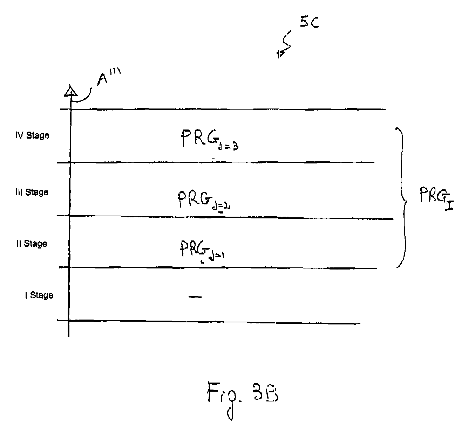

Advantageously, for visage treatment a protocol PRGᵢ is selected among a plurality N of predefined protocols PRG_{N} (figure 3A), while for the treatment of PEFS at least another protocol PRGⱼ is selected among a plurality I of predefined protocols of protocols PRG_{I} (figure 3B).

In other words, the protocols for visage treatment are different from the protocols for PEFS treatment.

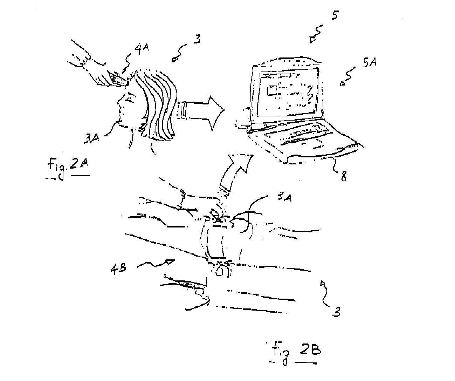

With reference to figures 2A and 2B, the first means 4 comprise a device 4A and a thermographic plate 4B.

In particular, the device 4A is suitable to measure characteristic data A such as the hydration data A' and/or the skin elasticity data A".

It is to be noted that the device 4A for detecting such characteristic data A' and/or A" should be arranged in contact with the dermis of the subject 3.

In a preferred embodiment, the hydration data A' are deduced by means of a device which is different from the one able to deduce the characteristic elasticity data A".

Such devices 4A are well known in the prior art and thus they will not be discussed in detail in the field of the present disclosure.

The thermographic plate 4B allows to measure the characteristic data A"' which are relative to the stage of the PEFS.

The stages of the PEFS, and thus the characteristic data A"', can be subdivided in four classes. Each class specifies the particular stage of PEFS, that is: I° = normal, II°= edematous, III° = fibrous with micronodules, IV° = fibrous with macronodules.

Also the thermographic plate 4B is a tool which is known in the prior art of the sector and thus it will not be discussed in detail in the field of the present invention.

In a further embodiment, the first means 4 which are suitable to detect the characteristic data A of the status of the skin 3A of the subject 3 may comprise litmus papers (not shown in the figures) for measuring the pH grade of the skin 3A of the subject 3, Wood light (not shown in the figures), sebometric devices (not shown in the figures), etc.

It is useful to consider that further characteristic data A"" can be detected, such as those herebelow listed:
- data of the skin ageing;
- data inherent to the health state of the subject;
- social food habits.

Said characteristic data A"" can be useful for the purposes of the cosmetic treatment.

The apparatus 6, in a preferred embodiment of the present invention, consists of an apparatus for performing the electroporation.

Such apparatuses are commercially known and thus the way they operate will not be described in the following.

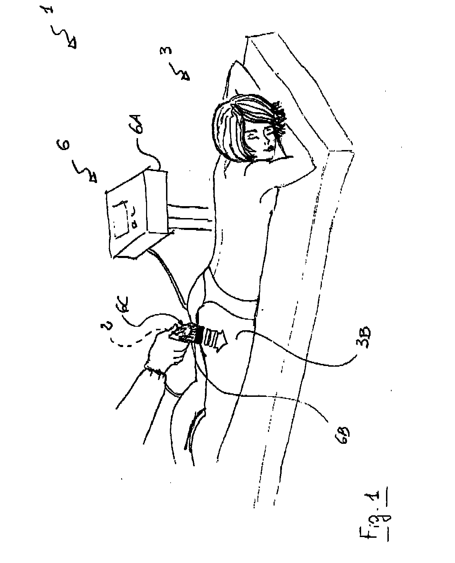

In the context of the present disclosure, it is useful to detect that the apparatus 6 comprises (figure 1):
- a principal unit 6A suitable to supply appropriate electric currents in order to generate micro-channels in the selected zone of the dermis of the subject 3;
- a handle 6B electrically connected to the principal unit 6A and provided with a reservoir 6C suitable to contain a predetermined amount of the cosmetic composition 2.

With reference to figures 3A and 3B, it is noted that the second means 5, in a first embodiment of the present invention, consist of a first synoptic table 5B and, optionally, of a second synoptic table 5C.

In particular, as it can be inferred from the embodiment illustrated in figure 3A, the first synoptic table 5B can be implemented by means of:
- an area 10 wherein the plurality N of predefined protocols PRG_{N} are arranged as a matrix;
- an abscissa axis indicating the characteristic data A', that is the skin hydration values of the subject 3, said axis providing four ranges, which are "LOW", "MEDIUM-LOW", "MEDIUM-HIGH" and "HIGH";
- an ordinate axis indicating the characteristic data A", that is the skin elasticity values of the subject 3, said axis providing three ranges, which are "LOW", "MEDIUM" and "HIGH".

Thus the area 10 is subdivided in twelve different cells, that is the number N of predefined protocols PRG_{N} for visage treatment is thus equal to twelve.

Advantageously, the synoptic table 5B allows to select a protocol PRGᵢ (with 1<i<N=12) among the plurality N of predefined protocols PRG_{N}, by combining values of said two characteristic data A' and A".

In other words, such a synoptic table 5B allows to select the most suitable protocol in function of the characteristic data A' and A" collected by the first means 4A.

For example, if the characteristic data deduced by the means 4A provide the following values A'="MEDIUM-HIGH" and A"="HIGH", the synoptic table 5B individuates the protocol PRGᵢ₌₉, that is a protocol for a tonic and hydrated skin.

With reference to the embodiment illustrated in figure 3B, the second synoptic table 5C can be implemented by means of:
- an area 11 wherein further predefined protocols PRG_{I} are arranged;
- an ordinate axis indicating the characteristic datum A"', that is the PEFS stages of the subject 3.

The area 11 is thus subdivided in four different zones, that is, the number I of predefined protocols PRG_{I} for PEFS treatment is thus equal to four.

However, it is to be noted that for the I° stage of the PEFS no protocol is provided. Therefore it means that the actual number I of predefined protocols PRG_{I} for PEFS treatment is equal to three.

Advantageously, the synoptic table 5C allows to select a protocol PRGⱼ (with 1<j<I=3) among the plurality I of predefined protocols PRG_{I}, starting from characteristic data A"'.

For example, if the characteristic data deduced by the means 4B provide the value A"'=III° stage, the synoptic table 5C individuates the protocol PRGⱼ₌₂, that is a protocol for a fibrous PEFS with micronodules.

Therefore, it is possible to select the most suitable protocol for treating the visage (PRGᵢ) and/or the PEFS (PRGⱼ) by using, respectively, synoptic tables 5B and 5C, in such a way to facilitate the operator (aesthetician) in selecting which protocols PRGᵢ and/or PRGⱼ should be administered to the subject 3 in function of the skin status of the subject.

Once the most suitable protocol PRGᵢ and/or PRGⱼ has been selected, it is possible to administer such a protocol to the subject 3, by means of the apparatus 6 for performing the electroporation.

It should be noted that each protocol PRGᵢ and/or PRGⱼ individuates a sequence of treatments which the skin 3A of the subject should undergo. Said sequence may include a plurality of treatments to be performed at home which are alternated to sessions at the beauty shop, by using one or several combinations of the cosmetic composition 2.

In particular, it is useful to notice that the protocol PRGᵢ and/or PRGⱼ which is individuated by means of the inventive system just described can advantageously comprise a follow-up step, during which the subject is given a kit of natural products allowing to continue the treatment at home. Such natural products have the same specificities as the natural products used by the beauty shop since the natural active principles are the same, obviously comprising different excipients in order to make them suitable for a manual application.

Referring now to figures 2A and 2B, it is noted that the second means 5 can comprise a software program 5A which is suitable to be executed by a computer 8.

Said software program 5A can be an alternative to the synoptic tables 5B, 5C previously described or it can be used in combination with such synoptic tables 5B, 5C.

Advantageously, the software program 5A can further help the operator in the step of selecting the most suitable protocol PRGᵢ and/or PRGⱼ for the skin status of the subject 3.

In fact, according to ways which are known in the prior art (illustrated with an arrow in figures 2A and 2B), the characteristic data A', A" and/or A"' are transferred in the computer 8 where the software program 5A resides.

Advantageously, the software program 5A comprises a memory area wherein the plurality of predefined protocols PRG_{N} (visage) and PRG_{I} (PEFS) are stored.

Such a software program 5A is able to receive as an input the characteristic data of the status of the skin 3A of the subject 3, such as for example the data relative to hydration A', elasticity A" and/or PEFS A"', and to combine one with the others in such a way to select from the memory area at least one protocol PRGᵢ and/or PRGⱼ among said plurality of predefined protocols PRG_{N} and/or PRG_{I}.

Advantageously, the software program 5A is able to select a protocol PRGᵢ (with 1<i<N=12) for visage treatment among said plurality of predefined protocols PRG_{N} and a protocol PRGⱼ (with 1<j<I=3) for PEFS treatment among said plurality of predefined protocols PRG_{I}, so as to provide the operator with the most suitable treatment for the visage and for the PEFS treatment.

In other words, the software program 5A allows to select the most suitable protocol for the skin status of the subject 3 and/or of the PEFS stage, in function of the collected characteristic data.

For example, if the characteristic data which are deduced by the means 4A and/or 4B provide the following values:
A'="MEDIUM-LOW"; A"="MEDIUM"; A"'=II° stage
it follows that the software program 5A produces the protocol PRGⱼ₌₅ as the result for the visage treatment, that is a protocol for normal and dehydrated skin, and the protocol PRGⱼ₌₁ for the PEFS treatment, that is a protocol for the edematous PEFS.

The software program 5A of the previously described kind can be implemented for example by means of an Excel-like spreadsheet.

Once the most suitable protocol PRGᵢ and/or PRGⱼ has been selected, it is possible to administer such a protocol to the subject 3, by means of the apparatus 6 for performing the electroporation.

As previously described, each protocol PRGᵢ and/or PRGⱼ individuates a sequence of treatments which the skin 3A of the subject 3 should undergo. Such a sequence can comprise a plurality of treatments to be performed at home which are alternated to sessions at the beauty shop, by using one or several combinations of the cosmetic composition 2.

It can be easily guessed that the system 1 just illustrated helps the operator to select the most suitable protocol for treating visage and/or PEFS, while avoiding, or at most limiting, possible evaluation mistakes which the operator might have made with the known systems.

Examining now the method of administering the cosmetic composition which is performed by the system 1, whose components have been previously illustrated, it is to be noted that such method can be performed by an operator (aesthetician), who at first should deduce the characteristic data A of the status of the skin 3A of the subject 3.

In particular, the operator should deduce the characteristic data A' and A" of the skin status of the subject 3. The characteristic data A' and A" are deduced by using the devices 4A, that is the devices which are suitable to measure the values of hydration and/or elasticity, while the characteristic data A"' are deduced by using thermographic plates 4B.

If it is necessary to deduce further data about the skin status of the subject 3 the operator can use other devices such as litmus papers for measuring the skin pH grade of said subject, Wood light, sebometric devices, etc.

It is to be noted that the operator may complete the collected characteristic data with further data A"", such as skin ageing, sebometric data, skin pH data, etc.

At this point the operator can transfer the characteristic data A' and A" on the synoptic table 5B and, optionally, the data A"' on the synoptic table 5C.

In particular, in the case of the synoptic table 5B, the operator can combine said characteristic data A' and A" in order to select at least one protocol PRGᵢ (with 1<i<N=12) among said plurality N of predefined protocols PRG_{N}, so as to individuate the most suitable protocol for visage treatment.

For example if A'="LOW" and A"="LOW", the operator has got the indication for the selection of the protocol PRGᵢ₌₁, that is a protocol for very dehydrated slack skin.

In the case of the synoptic table 5C, the operator can individuate in function of the characteristic data A"' which protocol PRGⱼ (with 1<j<I=3) should be selected among the plurality I of predefined protocols PRG_{I}, so as to individuate the most suitable protocol for the PEFS treatment.

For example if the data A"' correspond to the fourth stage of PEFS, that is fibrous with macronodules, the operator has got the indication for selecting the protocol PRGⱼ₌₃.

Alternatively or in combination with the synoptic tables 5B and 5C, the operator can insert the characteristic data A', A" and/or A"' in the computer 8 wherein the software program 5A is stored (e.g. an Excel-like spreadsheet) which is able to compute and compare said characteristic data introduced in order to select a protocol PRGᵢ and/or PRGⱼ among said plurality of predefined protocols PRG_{N} and/or PRG_{I}.

Once the most suitable protocol PRGᵢ and/or PRGⱼ for the skin 3A of the subject 3 has been selected, the operator can generate at least one micro-channel in the epidermis region of the subject 3 to which a predefined amount of the dermatologically compatible cosmetic composition 2 is to be applied.

It is useful to notice that said step of generating at least one micro-channel in an epidermis region of the subject is performed by means of the apparatus 6 for performing the electroporation.

The method hereabove illustrated thus allows to individuate which is the most suitable protocol PRGᵢ and/or PROⱼ for the skin status of the subject 3.

The individuated protocol defines a sequence of treatments which the skin 3A of the subject 3 should undergo, said sequence comprising a plurality of treatments to be performed at home which are alternated to sessions at the beauty shop, by using one or several combinations of the dermatologically compatible natural cosmetic composition 2.

Moreover it is useful to notice that the protocol PRGᵢ and/or PRGⱼ, which has been individuated by means of the above described inventive method, can advantageously comprise a follow-up step wherein the subject is given a kit of natural products allowing to continue the treatment at home. Such natural products have the same specificities as the natural products used by the beauty shop since the natural active principles are the same, obviously comprising different excipients in order to make them suitable for a manual application.

Obviously a person skilled in the art, in order to satisfy contingent and specific needs, will be able to make numerous changes and variations to the above described configurations, which are moreover all comprised in the scope of the protection of the invention as it is defined by the following claims.

## Claims

1. A system for administering a cosmetic composition to a subject (3), comprising:
- first means (4, 4A, 4B) suitable to deduce characteristic data (A, A', A", A"') about the skin status of the subject (3);
- an apparatus (6) suitable to generate at least one micro-channel in an epidermis region of the subject (3) in order to facilitate the administration of a dermatologically compatible cosmetic composition through said epidermis region,
**characterized in that** it comprises:
- second means (5, 5A, 5B, 5C) suitable to select at least one protocol (PRGᵢ, PRGⱼ) among a plurality of predefined protocols (PRG_{N}, PRG_{I}) in function of said characteristic data (A, A', A", A"') which are deduced by said first means (4, 4A, 4B);
wherein said dermatologically compatible cosmetic composition (2) is selected in function of said protocol (PRGᵢ, PRGⱼ) and comprises a dermatologically compatible natural product and at least one excipient.

2. The system for administering according to claim 1, wherein said dermatologically compatible natural product is a natural product which is selected between one or several phytocomplexes or derivates thereof.

3. The system for administering according to claims 1 to 2, **characterized in that** said first means (4, 4A, 4B) are selected from the group comprising devices for measuring elasticity and/or hydration (4A), thermographic plates (4B) for measuring skin temperature, sebometric devices, litmus papers for measuring skin pH grade of said subj ect.

4. The system for administering according to claims 1 to 3, **characterized in that** the second means (5, 5A, 5B, 5C) comprise a first synoptic table (5B) having a first group (PRG_{N}) of said plurality of predefined protocols (PRG_{N}, PRG_{I}), each of which can be individuated by combining at least two characteristic data (A', A") about the skin status of the subject (3), in order to select a protocol for visage treatment (PRGᵢ) from said first group (PRG_{N}) of said plurality of predefined protocols (PRG_{N}, PRG_{I}).

5. The system for administering according to claim 4, **characterized in that** the second means (5, 5A, 5B, 5C) comprise a second synoptic table (5C) having a second group (PRG_{I}) of said plurality of predefined protocols (PRG_{N}, PRG_{I}), each of which can be individuated by means of at least another characteristic datum (A"') about the skin status of the subject (3), in order to select a protocol for the PEFS treatment (PRGⱼ) from said second group (PRG_{I}) of said plurality of predefined protocols (PRG_{N}, PRG_{I}).

6. The system for administering according to claims 1 to 5, **characterized in that** the second means (5, 5A, 5B, 5C) comprise a software program (5A) suitable to be executed by a computer (8), said software program (5A) comprising a memory area wherein said plurality of predefined protocols (PRG_{N}, PRG_{I}) are stored, said software program (5A) being suitable to receive as an input said characteristic data (A', A", A"') about the skin status of the subject (3) in order to select from said memory area at least one protocol (PRGᵢ, PRGⱼ) of said plurality of predefined protocols (PRG_{N}, PRG_{I}).

7. The system for administering according to claim 6, **characterized in that** said software program (5A) is suitable to select a protocol for visage treatment (PRGᵢ) from a first group (PRG_{N}) of said plurality of predefined protocols (PRG_{N}, PRG_{I}) and/or a protocol for the PEFS treatment (PRGⱼ) from a second group (PRG_{I}) of said plurality of predefined protocols (PRG_{N}, PRG_{I}).

8. The system for administering according to anyone of the preceding claims, **characterized in that** said apparatus (6) which is suitable to generate at least one micro-channel is an apparatus for performing electroporation.

9. The system for administering according to anyone of the preceding claims, **characterized in that** each protocol (PRGᵢ, PRGⱼ) of said plurality of predefined protocols (PRG_{N}, PRG_{I}) individuates a sequence of applications which the skin of the subject should undergo, said sequence comprising a plurality of treatments to be performed at home which are alternated to sessions at the beauty shop, by using one or several combinations of the dermatologically compatible cosmetic composition (2).

10. A method for administering a cosmetic composition to one subject, comprising the steps of:
- deducing characteristic data (A, A', A", A"') about the skin status of the subject (3);
- selecting at least one protocol (PRGᵢ, PRGⱼ) among a plurality of predefined protocols (PRG_{N}, PRG_{I}) in function of said characteristic data (A, A', A", A"') about the skin status of the subject (3);
- selecting a dermatologically compatible cosmetic composition (2) in function of said protocol (PRGᵢ, PRGⱼ);
- generating at least one micro-channel in an epidermis region of the subject (3);
- applying topically a predefined amount of said dermatologically compatible cosmetic composition (2) to said epidermis region, said dermatologically compatible cosmetic composition (2) comprising a dermatologically compatible natural product and at least one excipient.

11. The method for administering according to claim 10, **characterized in that** said step of deducing the characteristic data (A, A', A", A"') about the skin status of the subject (3) comprises the further step of applying to the skin of the subject (3) devices for measuring elasticity and/or hydration (4A), thermographic plates (4B), litmus papers for measuring the pH grade, sebometric devices.

12. The method for administering according to claims 10 to 11, **characterized in that** said step of selecting said at least one protocol (PRGᵢ, PRGⱼ) comprises the further step of reporting a first group of characteristic data (A', A") of said characteristic data (A, A', A", A"') on a first synoptic table (5B) in order to select a protocol (PRGᵢ) for the visage treatment from a first group (PRG_{N}) of said plurality of predefined protocols (PRG_{N}, PRG_{I}) and/or comprises the further step of reporting a second group of characteristic data (A"') of said characteristic data (A, A', A", A"') on a second synoptic table (5C) in order to select a protocol (PRGⱼ) for the PEFS treatment from a second group (PRG_{I}) of said plurality of predefined protocols (PRG_{N}, PRG_{I}).

13. The method for administering according to claims 10 to 12, **characterized in that** said step of selecting said at least one protocol comprises the further step of introducing said characteristic data (A, A', A", A"') in a computer (8) provided with a software program (5A) and/or reporting a group of characteristic data (A', A") of said characteristic data (A, A', A", A"') on a first synoptic table (5B).

14. The method for administering according to claim 13, **characterized in that** said software program (8) is suitable to select at least one protocol for the visage treatment (PRGᵢ) from a first group (PRG_{N}) of said plurality of predefined protocols (PRG_{N}, PRG_{I}) and/or one protocol for the PEFS treatment (PRGⱼ) from a second group (PRG_{I}) of said plurality of predefined protocols (PRG_{N}, PRG_{I}).

15. The method for administering according to claims 10 to 14, **characterized in that** each protocol (PRGᵢ, PRGⱼ) of said plurality of predefined protocols (PRG_{N}, PRO_{I}) individuates a sequence of treatments which the skin of the subject (3) should undergo, said sequence comprising a plurality of treatments to be performed at home which are alternated to sessions at the beauty shop, by using one or several combinations of the dermatologically compatible cosmetic composition (2).

16. The method for administering according to claim 10 to 15, **characterized in that** said step of generating at least one micro-channel in an epidermis region of the subject is performed by means of an apparatus (6) for performing electroporation.

17. The method for administering according to claims 10 to 16, **characterized in that** said dermatologically compatible cosmetic composition (2) is a dermatologically compatible natural product selected from one or several phytocomplexes or derivates thereof.
